# EUROPEAN PATENT APPLICATION

(11) **EP 3 406 713 A1**
(43) Date of publication of application: **28.11.2018**
(21) Application number: 17774638.5
(22) Date of filing: 23.03.2017
(51) Int. Cl.: C12N 5/077, C12M 1/00, C12M 1/22, C12M 3/00, C12M 3/04

(54) **METHOD AND DEVICE FOR KEEPING SHAPE OF FRAGILE MATERIAL IN LIQUID**

(30) Priority: 28.03.2016 JP 2016063992
(71) Applicant: Terumo Kabushiki Kaisha, Tokyo 151-0072 (JP)
(72) Inventor: TAKEUCHI, Ryohei, Ashigarakami-gun Kanagawa 259-0151 (JP); KIKUCHI, Minori, Ashigarakami-gun Kanagawa 259-0151 (JP); NOGUCHI, Eri, Ashigarakami-gun Kanagawa 259-0151 (JP); YORI, Kouichirou, Ashigarakami-gun Kanagawa 259-0151 (JP)
(74) Representative: Prüfer & Partner mbB Patentanwälte · Rechtsanwälte
(86) International application number: PCT/JP2017/011628
(87) International publication number: WO 2017/170097

(57) **Abstract**

Provided are a method for allowing a vulnerable object to retain its form in a liquid, and a device and its constituents to put the method into practice.

A method for allowing a vulnerable object to retain its form in a liquid held in a container, the method including: covering the surface of the liquid with a molten sealing medium that melts and solidifies according to temperature change; and solidifying the sealing medium covering the liquid surface.

A device that allows a vulnerable object to retain its form in a liquid, the device including: a container to hold the vulnerable object therein; a sealing medium that melts and solidifies according to temperature change; and an optional lid for the container, with the sealing medium in a solidified state adhering to the container and/or the lid.

An annular member capable of adhering to an edge of a container holding therein a vulnerable object, the annular member having an annular groove to support therein a sealing medium that melts and solidifies according to temperature change, with the annular groove having an inner wall and an outer wall such that the former is lower in height than the latter.

## Description

### Technical Field

The present invention relates to a method that allows a vulnerable object to retain its form in a liquid, and also to a device and its constituents to put the method into practice.

### Background Art

The recent revolutionary method for the treatment of heart disease is not yet complete to cope with severe cardiac failure. A common treatment for cardiac failure is by internal therapy that relies on β-blocker or ACE inhibitor. For severe cardiac failure, however, the internal therapy is replaced by substitutional therapy, or surgical therapy, such as heart transplantation or use of an auxiliary artificial heart.

The severe cardiac failure that needs the surgical therapy mentioned above results from various causes, such as advanced valvular disease, hyper myocardial ischemia, acute cardiac infarction and complication thereof, acute myocarditis, and chronic cardiac failure and its acute animus due to ischemic cardiomyopathy (ICM) and dilated cardiomyopathy (DCM).

The cause and severity of disease determine the selection of therapeutic regimen, such as valve formation or replacement, coronary artery by-pass, left ventricular formation, and mechanical auxiliary circulation.

It has been believed that the above-mentioned cardiac failure which results from an extreme decrease in the left ventricular function due to ICM or DCM can be treated effectively only by the substitutional therapy based on heart transplantation or artificial heart. Unfortunately, the substitutional therapy for patients of serious cardiac failure is not necessarily universally useful on account of the chronic donor shortage, the necessity for continuous immune suppression, and the occurrence of complication.

On the other hand, there has recently arisen a new idea that problems with the therapy for serious cardiac failure should be tackled by the regenerative medicine which is currently developing.

The serious cardiac infarction leads to cardiac failure through the dysfunction of cardiac muscle cells, the proliferation of fibroblasts, and the fibrosing of interstitial tissues. The cardiac failure advances to damage cardiac muscle cells, giving rise to apoptosis. The cardiac muscle cells in this state rarely undergo cell division, which leads to a decrease in the number of cardiac muscle cells and a more decreased cardiac function.

It is considered that the patient of serious cardiac failure will have his/her cardiac function recovered effectively by cell transplantation. In fact, this new method is being put into clinical use with the help of autoskeletal muscle cells.

One of such examples has recently been disclosed, which is concerned with a cell culture and a method for production thereof (Patent Document 1). According to this disclosure, the cell culture has a three-dimensional structure suitable for application to a heart and is composed of cells derived from any other part of the body than the cardiac muscle. The cell culture is obtained with the help of temperature responsive culture dishes devised by the tissue engineering.

The sheet-shaped cell culture mentioned above needs to be kept in a liquid held in a container, and the container needs to be transferred to the place where the sheet-shaped cell culture is used later. For stable transportation of the sheet-shaped structured object, there has been developed a variety of methods and tools (Patent Documents 2 and 3).

Because of its inherently poor physical strength, the sheet-shaped cell culture mentioned above is vulnerable to wrinkling and breakage which occur when the surface of the liquid for storage waves. This gives rise to a need for any means for the sheet-shaped cell culture to retain its form in the storage liquid. There has been developed a method for preventing the liquid surface from waving (Patent Document 4). This method includes incorporating the storage liquid for the sheet-shaped structured object with another liquid that forms a liquid-liquid interface between them in the container closed with a lid.

### Prior Art Document

### Patent Documents

Patent Document 1: JP 2007-528755A
Patent Document 2: JP 2003-070460A
Patent Document 3: JP 4749155B2
Patent Document 4: JP 2013-192467A

### Summary of Invention

### Technical Problems

Under the circumstances mentioned above, the present inventors attempted to develop means to allow a vulnerable object, such as sheet-shaped structured object, to retain its form in a liquid. Unfortunately, however, they found that the container holding the liquid cannot be completely closed with the lid, with air remaining between the lid and the liquid surface. Such remaining air results in the waving of the liquid. Moreover, in the case where the liquid containing the sheet-shaped structured object is covered with another liquid, the vulnerable sheet-shaped structured object fluctuates as the second liquid waves. In order to tackle the foregoing problems, the present inventors propose herein a method and a device and constituents thereof, which are intended for a vulnerable object to retain its form in a liquid.

### Technical Solution

In order to tackle the foregoing problems, the present inventors carried out extensive researches. In the course of their research work, they found that it is necessary to cover the liquid surface with a solid so that the liquid containing the vulnerable object escapes movement. Further researches suggested that the liquid surface can be covered with a solid more adequately if the liquid surface is covered with a sealing medium in a molten state and subsequently the molten sealing medium is solidified. This finding led to the present invention.

Thus, the present invention is concerned with what is defined in the following paragraphs [1] to [7].
[1] A method for allowing a vulnerable object to retain its form in a liquid held in a container, the method including: covering the surface of the liquid with a molten sealing medium that melts and solidifies according to temperature change; and solidifying the sealing medium covering the liquid surface.
[2] The method as defined in paragraph [1] above, in which the vulnerable object is a sheet-shaped cell culture of skeletal myoblasts.
[3] The method as defined in paragraph [1] or [2], in which the sealing medium has a melting point of 0°C to 45°C and a solidifying point of 0°C to 45°C.
[4] The method as defined in any one of paragraphs [1] to [3], in which the sealing medium includes at least one selected from the group consisting of fatty ester, gelatin, polysaccharide, and polymeric compound.
[5] A device that allows a vulnerable object to retain its form in a liquid, the device including: a container to hold the vulnerable object therein; a sealing medium that melts and solidifies according to temperature change; and an optional lid for the container, with the sealing medium in a solidified state adhering to the container and/or the lid.
[6] The device as defined in paragraph [5], in which the container holding therein the vulnerable object has a groove to fix the sealing medium, with the groove having an inner wall and an outer wall such that the former is lower in height than the latter.
[7] An annular member capable of adhering to an edge of a container holding therein a vulnerable object, the annular member having an annular groove to support therein a sealing medium that melts and solidifies according to temperature change, with the annular groove having an inner wall and an outer wall such that the former is lower in height than the latter.

### Advantageous Effects

The present invention produces an effect of efficiently covering the liquid surface owing to the sealing medium that melts and solidifies in response of temperature change. Moreover, it produces an effect of efficiently preventing the liquid from moving owing to the sealing medium that solidifies on the liquid surface. It also produces an effect of protecting the vulnerable object in the liquid from contamination because the liquid is isolated from the outside air.

The present invention is particularly effective in preventing the vulnerable object from wrinkling, breaking, and deforming when its container is moved, thereby keeping it physically stable.

Moreover, the present invention provides a method and a device and constituents thereof, which are inexpensive and simple, for the vulnerable object to retain its form in the liquid.

### Brief Description of Drawings

[Fig. 1]

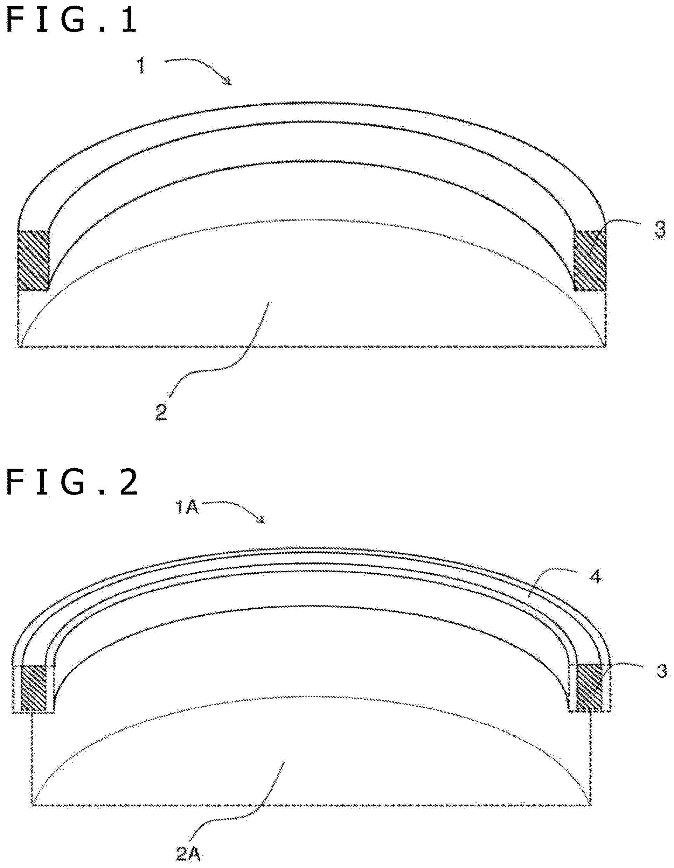

   Fig. 1 is a sectional perspective view depicting a device 1 pertaining to a first embodiment of the present invention.
[Fig. 2]
   Fig. 2 is a sectional perspective view depicting a device 1A pertaining to a second embodiment of the present invention.
[Fig. 3]

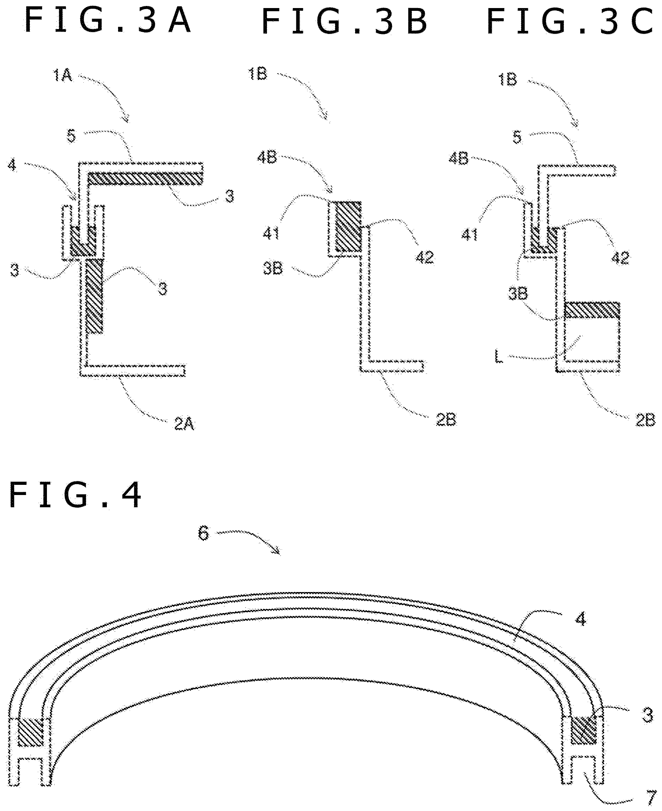

   Figs. 3A to 3C are schematic sectional views depicting modified examples of the device depicted in Fig. 2.
[Fig. 4]
   Fig. 4 is a sectional perspective view depicting a member pertaining to a third embodiment of the present invention.
[Fig. 5]

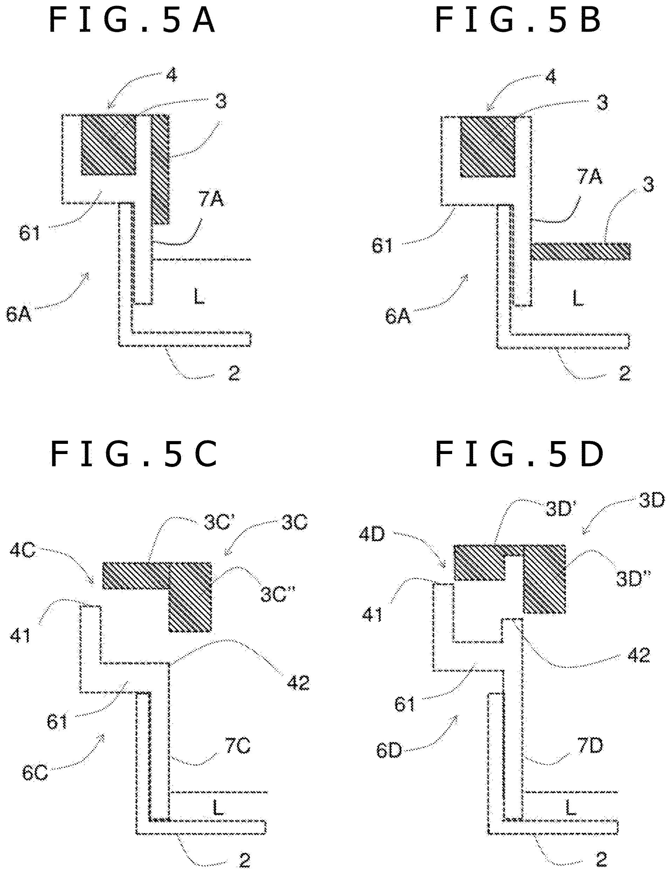

   Figs. 5A to 5D are schematic sectional views depicting modified examples of the member depicted in Fig. 4.
[Fig. 6]

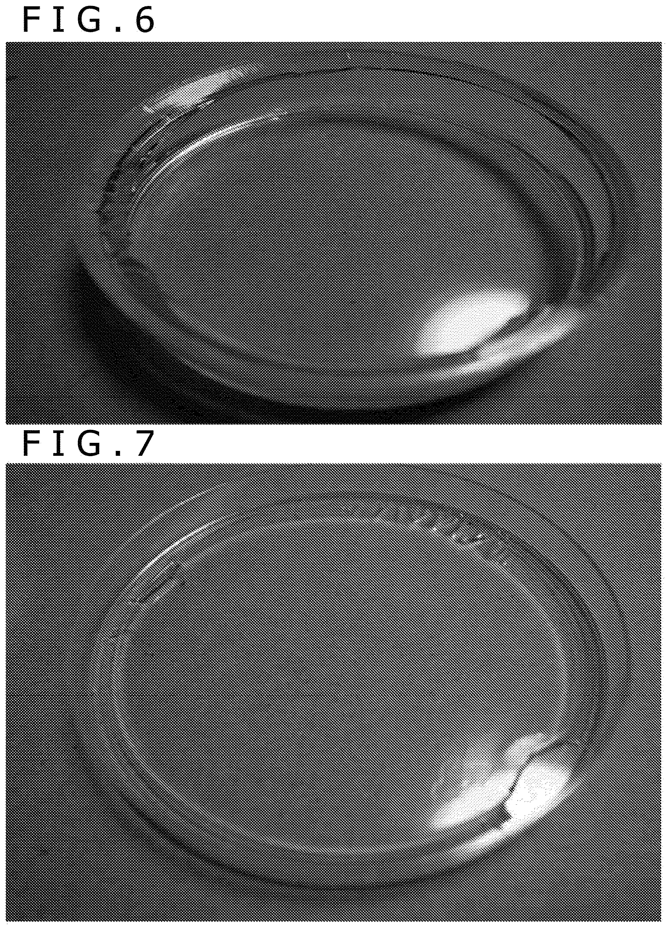

   Fig. 6 is a photograph showing a gelled gelatin which adheres to the inner side surface of a Petri dish.
[Fig. 7]
   Fig. 7 is a photograph showing a gelled gelatin which adheres to the inner surface of a lid.
[Fig. 8]

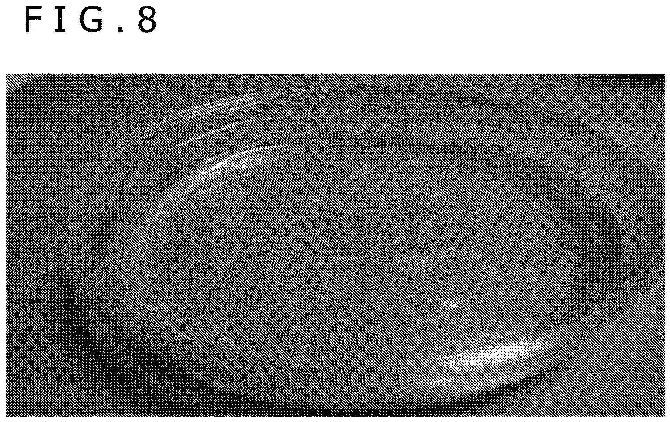

   Fig. 8 is a photograph showing a gelled gelatin that covers water in a Petri dish.

### Modes for Carrying Out the Invention

One aspect of the present invention covers a method for allowing a vulnerable object to retain its form in a liquid held in a container, the method including: covering the surface of the liquid with a molten sealing medium that melts and solidifies according to temperature change; and solidifying the sealing medium covering the liquid surface.

The term "vulnerable object" as used in this specification denotes any object which has such low physical strength as to deform or break when the liquid moves. Such objects include one which has a thin-walled part, one which takes on a belt-shaped form, and one which takes on a sheet-shaped form. Examples of the sheet-shaped object unrestrictedly include sheet-shaped structured objects, e.g., filmy tissue formed on a flat film from any material derived from a living organism such as sheet-shaped cell culture, and various kinds of film formed from plastic, paper, woven cloth, unwoven cloth, metal, polymer, lipid, and the like. Preferable among them are the ones which hardly decompose or disintegrate in a liquid. The sheet-shaped structured object may vary in shape (polygon, circle or the like), diameter, width, thickness, etc. Moreover, the sheet-shaped structured object in the present invention may be of monolayer or multilayer. The multilayered object may have layers joined together or separated from one another. The term "joined" does not necessarily mean that overlapped parts of the layers are entirely joined to one another.

The term "sheet-shaped cell culture" as used in this specification denotes a cell culture which resembles a sheet having cells joined together. The sheet-shaped cell culture typically consists of one layer of cells, but it may consist of two or more layers of cells. The cells may join together directly or indirectly with a substance existing among them. Such an intervening substance unrestrictedly includes any substance that is able to join cells together at least physically (mechanically); an example is an extracellular matrix. The intervening substance should preferably be one which is derived from cells, especially those cells which constitute the cell culture. These cells are joined together at least physically (mechanically) and may be further joined together functionally, that is, chemically or electrically.

The sheet-shaped cell culture involved in the present specification is comprised of any cells capable of forming the above-mentioned structure. Examples of such cells unrestrictedly include adherent cells. The adherent cells include adherent somatic cells and stem cells. The somatic cells include cardiac muscle cells, fibroblasts, epithelial cells, endothelial cells, hepatic cells, pancreatic cells, nephrocytes, adrenal cells, periodontal membrane cells, gingival cells, periosteal cells, dermal cells, synovial cells, and cartilage cells. The stem cells include myoblasts, tissue stem cells such as cardiac stem cells, embryonic stem cells, pluripotent stem cells such as iPS (induced pluripotent stem) cells, and mesenchymal stem cells. The somatic cells may be those which are obtained by differentiation from stem cells, especially iPS cells. The cells capable of forming the sheet-shaped cell culture unrestrictedly include myoblasts (e.g., skeletal myoblasts), mesenchymal stem cells (e.g., those which are derived from marrow, fat tissues, peripheral blood, skin, hair root, muscle tissue, endometrium, placenta, and cord blood), cardiac muscle cells, fibroblasts, cardiac stem cells, embryonic stem cells, iPS cells, synovial cells, chondrocytes, epithelial cells (e.g., oral mucosa epitheliocytes, retinal pigment epitheliocytes, and nasal mucosa epitheliocytes), endothelial cells (e.g., blood endothelial cells), hepatocytes (e.g., hepatic mesenchymal cells), pancreatic cells (e.g., islet cells), nephrocytes, adrenal cells, periodontal membrane cells, gingival cells, periosteal cells, and dermal cells. In the present specification, the preferable cells are myoblasts, especially skeletal myoblasts, which form monolayered cell cultures.

It is possible to use cells derived from any living organism that can be cured with the help of cell culture. Such living organisms unrestrictedly include human, primate, dog, cat, pig, horse, goat, sheep, etc. The sheet-shaped cell culture may be prepared from one species of cells or more than one species of cells. According to a preferred embodiment of the present invention, the cell culture should be prepared from more than one species of cells in such a way that the major cells account for equal to or more than 65%, preferably equal to or more than 70%, and more preferably equal to or more than 75%, when the cell culture is prepared completely, in the case of skeletal myoblasts, for example.

The sheet-shaped cell culture used in the present invention may be a cultured tissue in a sheet form which is obtained by inoculating cells onto a scaffold (for cell culturing) and culturing; however, it should preferably be one which is composed solely of substances derived from the cells constituting the cell culture.

The sheet-shaped cell culture may be prepared by any known method.

According to one aspect of the present invention, the sheet-shaped cell culture is a sheet-shaped cell culture of skeletal myoblasts. It is so vulnerable as to break by its own weight even when a part of it is pinched. Thus, it cannot be transported in its isolated form, or it presents extreme difficulties in restoring its original form once it is folded. Consequently, keeping the sheet form in a liquid is of great significance.

In the present specification, the liquid in the container is composed of at least one component, which is unrestrictedly exemplified by such liquid as water, aqueous solution, non-aqueous solution, suspension, emulsion, and the like.

Moreover, in the present specification, the liquid may contain solids (derived from the scaffold), bubbles, and any other non-liquid components as long as the liquid is a fluid having fluidity as a whole.

The liquid in the container may be composed of any components which affect little the vulnerable object. In the case where the vulnerable object is a film composed of materials derived from a living organism, the components constituting the liquid in the container should preferably be ones which are compatible with a living organism from the standpoint of biological stability and long-term storage stability. Biological stability means the complete or substantial absence of undesirable reactions such as inflammatory, immunity, and intoxication to the living organism and cells. Examples of such components include water, physiological saline, physiological buffer solution (such as HBSS, PBS, EBSS, Hepes, and sodium bicarbonate), culture medium (such as DMEM, MEM, F12, DMEM/F12, DME, RPMI1640, MCDB, L15, SkBM, RITC80-7, and IMDM), sugar solution (such as sucrose solution and Ficoll-paque (registered trademark) PLUS), sea water, serum-containing solution, Renografin (registered trademark) solution, metrizamide solution, meglumin solution, glycerin, ethylene glycol, ammonia, benzene, toluene, acetone, ethyl alcohol, oil, mineral oil, animal oil, vegetable oil, olive oil, colloidal solution, liquid paraffin, turpentine oil, linseed oil, and castor oil.

In the case where the vulnerable object is a sheet-shaped cell culture, the liquid in the container should preferably be composed of components that ensure stable cell storage, contain minimal oxygen and nutrients essential for cell existence, and have an osmotic pressure low enough not to break cells. Examples of the components to meet these requirements unrestrictedly include physiological saline, physiological buffer solution (such as HBSS, PBS, EBSS, Hepes, and sodium bicarbonate), culture medium (such as DMEM, MEM, F12, DMEM/F12, DME, RPMI1640, MCDB, L15, SkBM, RITC80-7, and IMDM), and sugar solution (such as sucrose solution and Ficoll-paque (registered trademark) PLUS).

The liquid in the container is not specifically restricted in amount; a desirable amount is such that the solution level is high enough to prevent the vulnerable object from moving. For example, in the case where the sheet-shaped cell culture of skeletal myoblasts has a diameter of approximately 10 mm or 20 mm, the height of the liquid level should be 0.1 to 11 mm or 0.1 to 16 mm, respectively. In addition, the amount of the liquid should preferably be 1 to 100 mL from the standpoint of cooling the sealing medium that covers the liquid surface. The liquid should preferably be one which has a specific gravity smaller than that of the vulnerable object, so that the vulnerable object will not float on the liquid surface to come into contact with the sealing medium. Moreover, the specific gravity of the liquid should preferably be equal to that of the vulnerable object, so that the vulnerable object stably keeps its form without moving in the liquid when the container inclines.

In this specification, the term "temperature change" denotes the change in temperature which the sealing medium experiences upon heating and cooling; the term "melt" denotes the phenomenon that the sealing medium partly or entirely melts upon heating; and the term "solidify" denotes the phenomenon that the sealing medium in a molten state partly or entirely solidifies or semisolidifies. Also, in the case where the sealing medium is composed of those components which undergo sol-gel transformation due to temperature change, the term "melt" denotes the phenomenon that the sealing medium dissolves in gel and the term "solidify" denotes the phenomenon that the sealing medium becomes gel. Therefore, in this specification, the term "melting point" is synonymous with "gel dissolving temperature" and the term "solidifying point" is synonymous with "gelling temperature."

In this specification, the sealing medium includes one which is composed of a single component or one which is composed of two or more components. The sealing medium denotes any material which melts upon heating to cover in a molten state the liquid surface, and which solidifies upon cooling to form a sealing lid on the liquid surface. The sealing medium should preferably be composed of components stable to oxidation and hydrolysis. The sealing medium may be made stable to oxidation and hydrolysis by incorporation with any known stabilizers in the field, such as antioxidant, weathering agent, UV absorber, and hydrolysis inhibitor. In this way, the sealing medium in contact with the liquid is saved from oxidation and hydrolysis and the sealing medium which has solidified on the liquid surface isolates the liquid from the external air while keeping its properties. This realizes the long-term storage of the vulnerable object in the liquid.

In the case where the vulnerable object consists of cells or contains cells, the sealing medium should preferably be composed of components compatible with living organisms, which would cause no or little immune reaction, inflammatory reaction, or toxic reaction undesirable for living tissues and cells. Also, the sealing medium should preferably be one which melts and solidifies in response to temperature change to produce no adverse effects on cells. Any sealing medium suitable for the transportation, storage and long-term storage of the vulnerable object may be prepared from various components which would be adequately selected by those who are skilled in the art from the standpoint of the range of temperature change and the compatibility to living organisms.

The sealing medium is not specifically restricted in melting point and solidifying point. The melting point should be 0°C to 45°C, preferably 10°C to 30°C, and more preferably 15°C to 25°C. The solidifying point should be 0°C to 45°C, preferably 10°C to 30°C, and more preferably 15°C to 25°C. For the sealing medium to solidify at room temperature, it should preferably have a solidifying point ranging from 30°C to 37°C. Moreover, the sealing medium should preferably have a melting point ranging from 0°C to 37°C so that it gives off a minimum of latent heat of fusion that affects cells contained in the vulnerable object in the liquid.

The sealing medium should be used in such an amount as to cover the entire liquid surface, with its thickness being 1 to 20 mm, preferably 1 to 10 mm, more preferably 3 to 5 mm.

The sealing medium includes, for example, fatty acid ester (wax with a solidifying point of 35°C to 50°C, animal fat with a solidifying point of 25°C to 50°C, and vegetable fat), gelatin (with a gelling temperature of 15°C to 30°C), polysaccharide, and polymeric compound. The polysaccharide and polymeric compound are exemplified by pectin (25°C to 45°C), agar-agar (25°C to 45°C), carageenan (25°C to 60°C), cellulose (30°C to 40°C), gellan gum (30°C to 40°C), methyl cellulose (30°C to 55°C), xanthan gum + locust bean gum (35°C to 60°C), and curdlan (30°C to 60°C), with their respective gelling temperatures indicated in parentheses. The polymeric compound includes telechelic PEG and polyethylene which undergo sol-gel conversion owing to the hydrophobic mutual reaction. The sealing medium in the present invention may be used alone or in combination with one another. An adequate sealing medium will be selected by those who are skilled in the art.

According to the present invention, the sealing medium is not specifically restricted in specific gravity so long as it can be placed on the liquid holding the vulnerable object. The preferred specific gravity is smaller than that of the liquid in the container and that of the vulnerable object.

In the present specification, the sealing medium may be melted in any manner without specific restrictions; melting may be accomplished by direct heating or indirect heating. In other words, the sealing medium may be melted by exposing it to hot air or by heating the container to which the sealing medium is fixed.

In the present specification, there are no specific restrictions on the process of covering the liquid surface with the sealing medium in a molten state. A preferable process for the vulnerable object such as sheet-shaped cell culture of skeletal myoblasts consists of slowly dropping the sealing medium from a pipette. This process prevents the vulnerable object from being damaged. Another possible process consists of dropping the sealing medium along the inner side surface of the container. This process allows the sealing medium to be dropped slowly to the liquid surface. The sealing medium may cover the liquid surface entirely or partly.

In the present specification, there are no specific restrictions on the process of solidifying the sealing medium covering the liquid surface. The sealing medium may be solidified by direct cooling or indirect cooling in which case the container is cooled. The cooling may be accomplished by means of a cooling device or a liquid placed in the container, or by natural cooling with the container standing at room temperature. The solidified sealing medium covering the liquid surface prevents the liquid surface from shaking because the sealing medium has its periphery in the radial direction fixed to the inner side surface of the container.

The foregoing simple procedure by which the liquid in the container holding the vulnerable object is covered with the sealing medium in a molten state, which is subsequently solidified, prevents the vulnerable object in the liquid from disintegration when the container is moved. That is, the liquid which is incompressible does not move and hence does not apply any force to deform the vulnerable object so long as there is no gas between the liquid surface and the solidified sealing medium. In this case, the vulnerable object may float in the liquid in the container or may exist on the bottom of the container. The sealing medium in a molten state can properly cover the liquid surface even when the liquid height varies depending on the container.

The method according to one aspect of the present invention may include an additional step of removing the sealing medium after it has solidified. With the sealing medium removed, it is possible to take out the vulnerable object from the liquid when it is used. The step of removing the sealing medium may include a step of melting the sealing medium. This procedure allows the sealing medium to be easily removed. Further, the step of removing the sealing medium may include a step of melting the sealing medium and then adding the liquid to the container. This procedure increases the volume of the liquid in the container and causes the molten sealing medium to be pushed out of the container. Thus, the sealing medium can be removed easily.

In one aspect of the present invention, the liquid used to form the vulnerable object is removed and replaced by another liquid that supports the vulnerable object to be reserved. This procedure eliminates the necessity of mechanically taking out the vulnerable object by means of hands or tools, for transfer to another container. This procedure is useful particularly for vulnerable sheet-shaped cell cultures. The necessary steps to achieve the purpose include preparing a sheet-shaped cell culture in a culture vessel filled with a culture medium (solution), removing the culture medium, filling the culture vessel with a preserving liquid, and pouring the sealing medium into the culture vessel. The sheet-shaped cell culture that experiences these steps is saved from unnecessary contact with anything other than the liquid during a period from preparation to transfer, and to transplantation. This has only limited chances for damage and contamination.

A further aspect of the present invention covers a device that allows a vulnerable object to retain its form in a liquid, the device including: a container to hold the vulnerable object therein; a sealing medium that melts and solidifies in response to temperature change; and an optional lid for the container, with the sealing medium in a solidified state adhering to the container and/or the lid.

In the present specification, the container is not specifically restricted so long as it is capable of holding therein the vulnerable object, liquid, and sealing medium and is capable of tightly holding the liquid. Any container, including commercial ones, may be used. It may be one which is made of such material as polyethylene, polypropylene, Teflon (registered trademark), polyethylene terephthalate, polymethyl methacrylate, nylon 6,6, polyvinyl alcohol, cellulose, silicone, polystyrene, glass, polyacrylamide, polydimethylacrylamide, and metal (e.g., iron, stainless steel, aluminum, copper, and brass). The container should preferably have at least one flat bottom which helps maintain the shape of the vulnerable object. It includes, for example, Petri dish, cell culture dish, and cell culture bottle.

In the present specification, the container may have a lid which is placed on the container to cover the upper part of the container, thereby achieving tight sealing. Any lid, including commercial ones, may be used. In addition, the lid may be a discoid drop lid which has a smaller diameter than the inside diameter of the container so that it can be placed on the liquid surface. The lid may be one which is made of such material as polyethylene, polypropylene, Teflon (registered trademark), polyethylene terephthalate, polymethyl methacrylate, nylon 6,6, polyvinyl alcohol, cellulose, silicone, polystyrene, glass, polyacrylamide, polydimethylacrylamide, and metal (e.g., iron, stainless steel, aluminum, copper, and brass).

The device according to the present invention may include a container, a sealing medium, and a lid (optional). The container may or may not hold therein the vulnerable object and the liquid. The solidified sealing medium may be fixed to the container, or to the lid, or to both the container and lid. The device according to the present invention may be used in such a way that the container holds therein the liquid and the vulnerable object, the sealing medium which is fixed to the container and/or the lid is melted so that it covers the liquid surface, and the sealing medium covering the liquid surface is solidified. This procedure permits the liquid surface to be covered with the lid. Moreover, this procedure prevents air from entering the gap between the liquid surface and the lid. The result is that even when the container is shaken during transportation, the liquid in the container remains stable without waving and moving, the vulnerable object remains stable, and the liquid avoids contact with outside air. This allows the vulnerable object in the liquid to retain its shape without deformation and contributes to its long-term storage.

The preferable embodiments of the present invention will be described below with reference to the accompanying drawings.

### [First Embodiment]

The following is a description of a device 1 pertaining to the first embodiment of the present invention, the device 1 being depicted in Fig. 1, which is a sectional perspective view. Incidentally, the drawings in the present application depict various components in an exaggerated scale, differing from actual dimensions, to facilitate explanation.

It is noted from Fig. 1 that the device 1 pertaining to the first embodiment of the present invention includes a container 2 and a sealing medium 3. The container 2 is a round Petri dish having an opening. The sealing medium 3 assumes an annular shape corresponding to the shape of the inner side surface of the container 2 and fixes on the upper part of the inner side surface of the container 2. When the device 1 is put to use, the container 2 is allowed to hold therein a liquid and a vulnerable object and then heated so that the sealing medium 3 fixed to the container 2 melts. The sealing medium 3 in a molten state covers the liquid surface, and subsequently, the molten sealing medium 3 covering the liquid surface is allowed to solidify so that it forms a lid which covers the liquid surface.

According to the present embodiment, the sealing medium 3 adheres to the upper part of the container 2, so that it can cover the liquid held in the container 2 thereunder from above when it melts. Moreover, the sealing medium 3 adheres to the inner side surface of the container 2, so that the molten sealing medium 3 can slowly drip down along the inner side surface of the container 2. In addition, the sealing medium 3 assumes an annular shape, so that it can drip down uniformly in the circumferential direction of the container 2.

The foregoing embodiment, in which the sealing medium 3 adheres to the upper part of the container 2, is not intended for restrictions. That is, the sealing medium 3 may adhere to the bottom, or the lower part, of the container 2. The sealing medium 3 may take on any shape in conformity with the shape of the container 2. For example, it may assume a discoid shape that entirely covers the opening of the container 2. The discoid sealing medium 3 can drip on the entire liquid surface from above when it melts. In addition, the sealing medium 3 may adhere to the inner surface of the lid (not depicted) of the container 2 rather than the container 2 itself. In this case, the sealing medium 3 in a molten state covers the inner surfaces of the lid and the container 2 and the liquid surface, so that it fixes the lid and the container 2, thereby ensuring the tight sealing and the long-term storage of the vulnerable object. This is facilitated if the inner surface of the lid is inclined so that the sealing medium 3 in a molten state is led to the inner side surface of the container 2. As mentioned above, the sealing medium 3 will function as desired regardless of whether it is fixed to the container 2, to the lid, or to both the container 2 and the lid.

As mentioned above, the device 1 according to the first embodiment of the present invention is characterized in that the surface of the liquid in the container is properly covered and air is absent between the liquid surface and the sealing medium. This produces the effects of protecting the vulnerable object from moving even when the container shakes during transportation and the liquid in the container waves, and also preventing the liquid from coming into contact with external air. The foregoing effects permit the vulnerable object to be stored for a long period of time without deformation in the liquid.

### [Second Embodiment]

The following is a description of a device 1A pertaining to the second embodiment of the present invention, the device 1A being depicted in Fig. 2, which is a sectional perspective view. Incidentally, the same components as those of the device 1 of the first embodiment are indicated by the identical symbols, with their explanation omitted.

As depicted in Fig. 2, the device 1A pertaining to the second embodiment of the present invention includes a container 2A and the sealing medium 3. The container 2A has at its opening edge an annular groove 4 which permits a lid (not depicted) of the container 2A to fit in and also holds the sealing medium 3 that adheres to the inside thereof. When the device 1A is put to use, the container 2A is filled with a liquid and a vulnerable object is placed in the liquid, and the container 2A is closed with the lid which is placed on the groove 4 and heated subsequently. Upon heating, the sealing medium 3 melts to cause the lid to enter the inside of the groove 4 thereby pushing the sealing medium 3 out of the groove 4. The sealing medium 3 which has been pushed out drips down along the inner side surface of the container 2A to cover the liquid surface. The next step is to solidify the sealing medium 3 on the liquid surface and in the groove 4. This step causes the sealing medium 3 to simultaneously cover the liquid surface and fix the lid to the container 2A. This makes the container 2A to become tightly sealed.

According to this embodiment, the groove 4 has an annular shape; but this is not necessarily essential. The shape may vary in conformity with the shape of the lid. The groove 4 may be of annular shape, which makes the lid fit easily, in the case where the container 2A is a round Petri dish, with its opening and outer side surface covered by a round lid. The groove 4 may have any volume which is large enough to accommodate the sealing medium 3 that can fix the lid to the container 2A and cover the liquid surface.

As mentioned above, the device 1A pertaining to the second embodiment of the present invention is able to properly cover the surface of the liquid in the container and air does not enter between the liquid surface and the sealing medium. This produces the effects of protecting the vulnerable object from moving even when the container shakes during transportation and the liquid in the container waves, and also preventing the liquid from coming into contact with external air. The foregoing effects permit the vulnerable object to be stored for a long period of time without deformation in the liquid.

The device depicted in Fig. 2 may be modified as depicted in Figs. 3A to 3C, which are schematic sectional views. Incidentally, the same components as those of the device of the second embodiment are indicated by the identical symbols, with their explanation omitted.

### First modified example

As depicted in Fig. 3A, a device 1A pertaining to the first modified example of the present invention is characterized in that the sealing medium 3 adheres to the inside of the groove 4 of the container 2A, to the inner side surface of the container 2A, and to a lid 5. When the device 1A of the present invention is put to use, the container 2A is given a liquid and a vulnerable object and the lid 5 is placed in the groove 4 of the container 2A, and subsequently the resulting assembly is heated so that the sealing medium 3 melts. The molten sealing medium 3, which is on the inner side surface of the container 2A, drips down to the liquid surface from the outer side in the radial direction of the container 2A; the molten sealing medium 3 adhering to the lid 5 drips to the liquid surface from the inner side in the radial direction of the container 2A; and the molten sealing medium 3 in the groove 4 of the container 2 enters the gap between the groove 4 and the lid 5. The result is that the entire liquid surface is covered rapidly and the lid 5 and the container 2A are fixed together.

According to this modified example, the sealing medium 3 adheres to the inner side surface of the container 2A, the groove 4, and the lid 5. This structure may be modified such that the sealing medium 3 adheres to at least one of the inner side surface of the container 2A, the groove 4, and the lid 5. For example, even in the case where the sealing medium 3 adheres to the lid 5 only, the desired object will be achieved if the amount of the sealing medium 3 is properly adjusted so that the molten sealing medium 3 fills the groove 4 of the container 2A along the inner surface of the lid 5 and the molten sealing medium 3 overflows the groove 4 so as to entirely cover the liquid surface. Therefore, the lid 5 of the container 2A may have its inner surface so formed as to incline toward the side surface of the container 2A. This permits the sealing medium 3 to flow toward the groove 4 and the inner side surface of the container 2A.

### Second modified example

As depicted in Fig. 3B, a device 1B pertaining to the second modified example of the present invention is characterized in that a container 2B has a groove 4B on the edge thereof to which the lid 5 thereof fits, and the groove 4B has its inner wall 42 so formed as to exist along the inner side surface of the container 2B. In addition, the groove 4B is formed such that the inner wall 42 thereof is lower than the outer wall 41 thereof. The device 1B of the present invention is used in such a way that a sealing medium 3B to be fixed to the inside of the groove 4B is higher than the inner wall 42 of the groove 4B. The container 2B is given a liquid L and a vulnerable object, and the assembly is heated. The sealing medium 3B melts in the groove 4B, and the sealing medium 3B existing above the top of the inner wall 42 of the groove 4B drips down along the inner side surface of the container 2B to cover the surface of the liquid L (Fig. 3C). As the sealing medium 3B covering the liquid surface solidifies, the outer part in the radial direction of the sealing medium 3B adheres to the inner side surface of the container 2B, thereby making the liquid surface to hardly move. In addition, the sealing medium 3B keeps its lower side in the groove 4B, thereby fixing the lid 5 to the inside of the groove 4B.

The modified example mentioned herein is characterized in that the groove 4B is constructed such that the inner wall 42 is lower than the outer wall 41. The groove 4B may be constructed in this way entirely or partly. The object of this structure may be achieved, for example, if the groove 4B has a hole made at a certain part thereof which causes the inside of the groove 4B to communicate with the inner side surface of the container 2B.

As mentioned above, the device 1B according to this modified example permits the molten sealing medium 3B to be introduced into the container 2B from the groove 4B even if the lid 5 is not fit into the groove 4B. Therefore, the device according to the present invention does not necessarily include the lid.

Moreover, in the case where the lid is a discoid drop lid as mentioned above, it may be placed on the liquid surface, with the sealing medium fixed to at least one side thereof, and then the sealing medium is melted and solidified. This produces an advantage that the drop lid has its side surface fixed to the inner side surface of the container by the sealing medium so that the liquid surface is covered with a solid and the sealing medium has its amount saved. The drop lid may be made of a material which is harder than the solidified sealing medium. The resulting drop lid will hold the liquid surface more firmly so that the drop lid is sure to prevent the liquid from moving even when the container is inclined excessively.

The drop lid may be removed by breaking or melting the sealing medium. The procedure for removal may be made easy if the drop lid is provided with a notch, opening, tab, or the like. The opening may be one which can be opened and closed, so that it is closed when the drop lid is fixed to the container for the long-term storage of the vulnerable object in the liquid and it is opened for air to pass so that the drop lid is removed easily without the necessity of melting the sealing medium.

The foregoing demonstrates that the devices 1A and 1B pertaining to the modified examples of the present invention adequately cover the surface of the liquid in the container and prevent air from entering the gap between the liquid surface and the sealing medium. This produces the effect of protecting the liquid in the container from waving and exposing itself to external air and hence protecting the vulnerable object from moving even when the container experiences vibration during transportation. The effect mentioned above contributes to storing the vulnerable object stably for a long period of time, with its form maintained.

Another aspect of the present invention covers an annular member capable of adhering to an edge of a container holding therein a vulnerable object, the annular member having an annular groove to support therein a sealing medium that melts and solidifies according to temperature change, with the annular groove having an inner wall and an outer wall such that the former is lower in height than the latter.

### [Third Embodiment]

The following is a description of a member pertaining to the third embodiment of the present invention. Fig. 4, which is a sectional perspective view, depicts a member pertaining to the third embodiment of the present invention. Incidentally, the same components as those of the devices in the first and second embodiments are indicated by the identical symbols, with their explanation omitted.

It is to be noted from Fig. 4 that a member 6 pertaining to the third embodiment of the present invention is an annular member that can adhere to the edge of a container (not depicted) to hold therein a vulnerable object. The member 6 has the groove 4 to which a lid (not depicted) of the container fits, and the groove 4 holds the sealing medium 3 therein that adheres to it. In addition, the member 6 has a fitting part 7 formed therein which can firmly fix on the edge of the container. According to this embodiment, the fitting part 7 and the groove 4 take on an annular shape.

The member 6 is used in such a way that the member 6 has its fitting part 7 fitted to the edge of the container holding therein a liquid and the vulnerable object, the member 6 is firmly fixed to the container, and the lid is placed on the groove 4 of the member 6. Subsequently, the entire system is heated so that the sealing medium 3 melts and the lid enters the groove 4 to push out the sealing medium 3 to cover the liquid surface along the inner side surface of the member 6. The sealing medium 3 covering the liquid surface is cooled to solidify by the liquid in the container, with the result that the lid adheres to the container and the sealing medium 3 tightly closes the liquid in the container. The sealing medium 3 drips down onto the liquid surface along the inner side surface of the member 6 and hence it hardly adheres to the container. It is only necessary to take away the member 6 from the container in order to remove easily the sealing medium 3 from the liquid surface. It is also possible to remove the sealing medium 3 more easily if a hole for air to pass through is made in the solidified sealing medium 3. Such a hole for air to pass through may be made in the member 6 so as to open and close so that the sealing medium 3 can be removed easily without the necessity for making a hole in it. In this manner, the member 6 can be freely attached and detached to and from any commercial container and lid and permits the solidified sealing medium 3 to be removed easily from the liquid surface.

The member 6 may be one which is made of such material as polyethylene, polypropylene, Teflon (registered trademark), polyethylene terephthalate, polymethyl methacrylate, nylon 6,6, polyvinyl alcohol, cellulose, silicone, polystyrene, glass, polyacrylamide, polydimethylacrylamide, and metal (e.g., iron, stainless steel, aluminum, copper, and brass). The member 6 should preferably be flexible one from the standpoint of its easy attaching and detaching.

It has been demonstrated above that the member 6 pertaining to the third embodiment of the present invention properly covers the surface of the liquid in the container, thereby preventing air from entering the gap between the liquid surface and the sealing medium, with the result that the liquid in the container does not wave nor does it come into contact with external air and the vulnerable object in the liquid does not move even when the container experiences vibration during transportation. The foregoing effects permit the vulnerable object to be stored for a long period of time without deformation in the liquid.

The member depicted in Fig. 4 may be modified as depicted in Figs. 5A to 5D, which are schematic sectional views. Incidentally, the same components as those in the devices pertaining to the first and second embodiments and the member pertaining to the third embodiment are indicated by the identical symbols, with their explanation omitted.

### First modified example

As depicted in Fig. 5A, a member 6A pertaining to the first modified example of the present invention is characterized in that the sealing medium 3 is fixed in the groove 4 and onto the inner side surface of the member 6A and that a fitting part 7A is not an annular groove but takes on an annular wall that protrudes downward from the bottom surface of a base 61 of the member 6A. The fitting part 7A is formed so as to have its outer side surface in tight contact with the inner side surface of the container 2. The fitting part 7A is long enough for its lower end to dip into the liquid L held in the container 2. The sealing medium 3 melts on heating, and the sealing medium 3 in a molten state drips down along the inner side surface of the sealing medium 3 to reach the liquid surface and flow toward the center of the liquid surface. As the result, the sealing medium 3 has its circumferential edge fixed to the inner side surface of the member 6A (Fig. 5B). This arrangement makes it easy to remove the sealing medium 3 from the liquid surface by simply removing the member 6A from the container 2.

### Second modified example

It is depicted in Fig. 5C that a member 6C pertaining to the second modified example of the present invention does not have a sealing medium fixed to a groove 4C. According to this modified example, the groove 4C is not an annular groove but an annular wall that protrudes upward from the top of the base 61 of the member 6C. According to this modified example, the groove 4C is constructed such that the inner part (inner wall) 42 thereof is lower than the outer wall 41 thereof and is not protruding from the base 61. The member 6C pertaining to the present invention is put to use in such a way that the member 6C is fixed to the container (not depicted) and a sealing medium 3C in an annular shape is placed in the groove 4C and then heated. The sealing medium 3C to be employed in this modified example consists of an annular sealing medium 3C' that can be placed in the groove 4C of the member 6C and an annular sealing medium 3C" that can be placed on the inner side surface of the member 6C, which are integrally molded. The sealing medium 3C melts in the groove 4C and on the inner side surface of the member 6C, and drips down along the inner side surface of a fitting part 7C.

In this manner, the member 6 pertaining to the present invention may have the sealing medium 3 mounted later. Moreover, it is not necessary for the sealing medium 3 to be exactly adjusted in height with the inner part 42 and the outer wall 41 of the groove 4C, because the inner part 42 of the groove 4C does not protrude from the base 61. The modified example mentioned herein is characterized in that the fitting part 7C is approximately as long as the height of the inner side surface of the container 2. This permits the fitting part 7C to completely cover the inner side surface of the container 2. Thus, the sealing medium 3 can be led to the liquid surface along the inner side surface of the member 6C even in the case where the liquid level in the container 2 is low, as depicted in Fig. 5C.

In the present modified example, the member 6C may be used in such a way that the member 6C is fixed to the container, the annular sealing member 3C is placed in the groove 4C, and the lid (not depicted) is placed on the groove 4C. When the sealing medium 3C melts in this state, the lower end of the lid sinks into the sealing medium 3C' which is melting, and finally reaches the top of the groove 4C. When the lower end of the lid reaches the top of the groove 4C, that portion of the sealing medium 3C' which is in the outside of the lid has nowhere to go and stays between the lid and the outer wall 41 of the groove 4C. The sealing medium is solidified in this state so that the sealing medium staying between the lid and the outer wall 41 of the groove 4C fixes the lid to the groove 4C.

According to the present modified example, the groove 4C is not an annular groove which permits the lid to enter it. This structure may be applied to the grooves 4 and 4B depicted in Figs. 3A to 3C. In this case, for example, the lid may take on a discoid shape which has a smaller diameter than the outside diameter of the grooves 4, 4B, and 4C. This obviates the necessity of fitting the lid into the groove; it is only necessary to place the lid on the groove to achieve the desired object.

### Third modified example

In a member 6D pertaining to the third modified example of the member 6 of the present invention, as depicted in Fig. 5D, a sealing medium is not fixed in a groove 4D. A fitting part 7D protrudes downward from the lower surface of the base 61 of the member 6D and the fitting part 7D is longer than the height of the inner side surface of the container 2. This structure permits the fitting part 7D to entirely cover the inner side surface of the container 2. In this modified example, the groove 4D consists of two annular walls protruding upward from the upper surface of the base 61 of the member 6D. The two annular walls include an inner annular wall 42 and an outer annular wall 41 higher than the inner annular wall 42, and a sealing medium 3D is higher than the inner annular wall 42 and lower than the outer annular wall 41.

When the member 6D is put to use, it is fixed to the edge of the container 2 which holds therein a liquid and a vulnerable object and the sealing medium 3D in an annular shape is placed on the groove 4D and then heated. This modified example employs the sealing medium 3D which consists of an annular sealing medium 3D' and an annular sealing medium 3D" which are integrally molded, with the former being placeable on the groove 4D of the member 6D and the latter being placeable on the inner side surface of the member 6D. When the sealing medium 3D melts, the annular sealing medium 3D' positioned above the inner annular wall 42 and the annular sealing medium 3D" drip down along the inner side surface of the member 6D to reach the liquid surface. The lower portion of the sealing medium 3D' stays in the groove 4D, thereby properly fixing the lid fitting in the groove 4D**.**

As mentioned above, the sealing medium 3 may be formed arbitrarily in conformity with the shape of the member 6. For example, the sealing medium in an annular shape may be replaced by one in a discoid shape. When the sealing medium in a discoid shape melts, the outer part of the sealing medium may drip on the radially outer side of the liquid surface, and at the same time the inner part of the sealing medium may drip on the radially inner side of the liquid surface.

In addition, the fitting part 7D may be properly adjusted in length according to the height of the container and the height of the surface of the liquid in the container. The fitting part 7D is not specifically restricted in length; the length may range from 16 to 25 mm, preferably from 16 to 22 mm, and more preferably from 16 to 19 mm, for example.

It is noted from the foregoing that the modified examples of the present invention employ the member 6A, 6C, and 6D which can properly cover the surface of the liquid in the container, thereby preventing air from entering the gap between the liquid surface and the sealing medium, with the result that the liquid in the container does not wave nor does it come into contact with external air and the vulnerable object in the liquid does not move even when the container experiences vibration during transportation. The foregoing effects permit the vulnerable object to be stored for a long period of time without deformation in the liquid.

The foregoing demonstrates the devices and members pertaining to the embodiments 1 to 3 of the present invention. They are not intended to restrict the scope of the present invention. They may be properly modified and the modified products may be properly combined together to design the device and member differing in shape and structure, by those who are skilled in the art. For example, it will be possible to design the device 1 and the member 6 differing in shape and structure by properly combining the structures such as the grooves 4 and 4B and the sealing medium 3 of the device 1, and the grooves 4C and 4D and the sealing mediums 3C and 3D of the member 6.

The components used in the present invention may be substituted by arbitrary components which can function in the same way or may be added with arbitrary structure.

### [Example]

The present invention will be described below in more detail with reference to a typical example thereof, which is not intended to restrict the scope thereof.

### Example 1

A gelatin in a molten state was applied to the inner side surface of a Petri dish (Nunc (registered trademark)), 10 cm in diameter, and the inner surface of a lid for the Petri dish, and then the dish and lid coated with gelatin were cooled in a refrigerator for one hour to bring about gelation. The gelled gelatin adhering to the inner side surface of the dish is shown in Fig. 6, and the gelled gelatin adhering to the inner surface of the lid is shown in Fig. 7. The dish was then charged with water and closed by the lid, and heated at approximately 40°C until the gelatin melts. After the molten gelatin covered the water in the dish, the dish was refrigerated again for one hour to bring about gelation. It was found as shown in Fig. 8 that the gelled gelatin covered the water in the dish, thereby preventing the water in the dish from waving and moving.

### Description of Reference Symbols

- 1, 1A, 1B: Device
- 2, 2A, 2B: Container
- 3, 3B, 3C, 3D: Sealing medium
- 4, 4B, 4C, 4D: Groove
- 5: Lid
- 6, 6A, 6C, 6D: Member
- 7, 7A, 7C, 7D: Fitting part

## Claims

1. A method for allowing a vulnerable object to retain its form in a liquid held in a container, the method comprising:
covering the surface of the liquid with a molten sealing medium that melts and solidifies according to temperature change; and
solidifying the sealing medium covering the liquid surface.

2. The method as defined in claim 1, wherein the vulnerable object is a sheet-shaped cell culture of skeletal myoblasts.

3. The method as defined in claim 1 or 2, wherein the sealing medium has a melting point of 0°C to 45°C and a solidifying point of 0°C to 45°C.

4. The method as defined in any one of claims 1 to 3, wherein the sealing medium includes at least one selected from the group consisting of fatty ester, gelatin, polysaccharide, and polymeric compound.

5. A device that allows a vulnerable object to retain its form in a liquid, the device comprising:
a container to hold the vulnerable object therein;
a sealing medium that melts and solidifies according to temperature change; and
an optional lid for the container, with the sealing medium in a solidified state adhering to the container and/or the lid.

6. The device as defined in claim 5, wherein the container holding therein the vulnerable object has a groove to fix the sealing medium, with the groove having an inner wall and an outer wall such that the former is lower in height than the latter.

7. An annular member capable of adhering to an edge of a container holding therein a vulnerable object, the annular member having an annular groove to support therein a sealing medium that melts and solidifies according to temperature change, with the annular groove having an inner wall and an outer wall such that the former is lower in height than the latter.
